(19) Europäisches Patentamt
European Patent Office
Office européen des brevets

(11) Veröffentlichungsnummer: **0 254 158 B1**

(12) # EUROPÄISCHE PATENTSCHRIFT

(45) Veröffentlichungstag der Patentschrift: **29.01.92**

(51) Int. Cl.⁵: **C07D 239/48**, C07D 239/50

(21) Anmeldenummer: **87109987.5**

(22) Anmeldetag: **10.07.87**

(54) **Pyrimidinderivate und Verfahren zu ihrer Herstellung.**

(30) Priorität: **10.07.86 HU 285686**

(43) Veröffentlichungstag der Anmeldung:
**27.01.88 Patentblatt 88/04**

(45) Bekanntmachung des Hinweises auf die Patenterteilung:
**29.01.92 Patentblatt 92/05**

(84) Benannte Vertragsstaaten:
**AT BE CH DE ES FR GB GR IT LI NL SE**

(56) Entgegenhaltungen:
**GB-A- 2 032 434**
**GB-A- 2 102 800**

**JOURNAL OF HETEROCYCLIC CHEMISTRY, Band 15, Dezember 1978, Seiten 1529-1530, Hetero Corp.; J.M. McCALL et al.: "The reaction of 2,4-diamino-6-piperidinopyrimidine 3-oxide with acid anhydrides"**

(73) Patentinhaber: **RICHTER GEDEON VEGYESZETI GYAR R.T.**
**Gyömröi ut 19-21**
**H-1475 Budapest X(HU)**

(72) Erfinder: **Vedres, András, Dr.**
**Füredi u. 56**
**H-1144 Budapest(HU)**
Erfinder: **Szántay, Csaba, Dr.**
**Zsombolyai u.8**
**H-1113 Budapest(HU)**
Erfinder: **Stefk , Béla, Dr.**
**Orlay u. 2/b**
**H-1117 Budapest(HU)**
Erfinder: **Kreidl, János, Dr.**
**Pusztaszeri u. 51**
**H-1025 Budapest(HU)**
Erfinder: **Nemes, András, Dr.**
**Hank czi u. 11**
**H-1022 Budapest(HU)**
Erfinder: **Blask , Gábor, Dr.**
**Molnár Erik u. 21**
**H-1113 Budapest(HU)**
Erfinder: **Bogsch, Erik**
**Lidérc u. 40-42**
**H-1121 Budapest(HU)**

EP 0 254 158 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Máthé, Dénes**
**Eszék u. 13/15**
**H-1114 Budapest(HU)**
Erfinder: **Hegedüs, István**
**Balogh A. u. 7/b**
**H-1026 Budapest(HU)**
Erfinder: **Szuchovszky, Adrienn, Dr.**
**Páva u. 34**
**H-1094 Budapest(HU)**
Erfinder: **Mester, Tamás**
**Bors u. 56**
**H-1173 Budapest(HU)**

(74) Vertreter: **Beszédes, Stephan G., Dr. Patentanwalt**
**Münchener Strasse 80a Postfach 1168**
**W-8060 Dachau(DE)**

## Beschreibung

Die Erfindung betrifft neue Pyrimidinderivate und ein neues Verfahren zur Herstellung derselben sowie von bekannten anderen Pyrimidinderivaten.

Es sind bereits Pyrimidinderivate der allgemeinen Formel

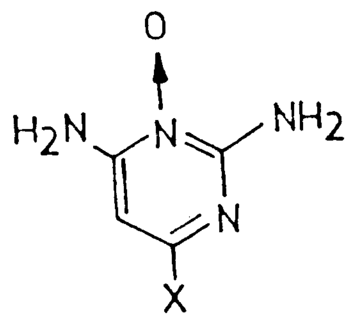

Id

bekannt, und zwar zum Teil (X = Chlor- oder Bromatom) aus der US-PS 3 644 364 und zum Teil (X = Arylsulfonyloxyrest) aus der HU-A-177 601 bzw. der ihr entsprechenden GB-A-2 032 434. Diese bekannten Verbindungen werden nach diesen Druckschriften aus den entsprechenden 2,6-Di-(amino)-pyrimidinderivaten der allgemeinen Formel

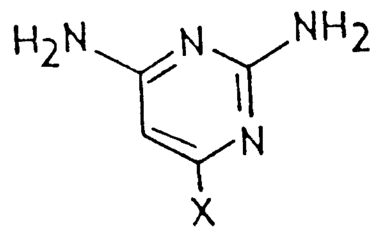

II ,

worin

X die vorstehend angegebenen Bedeutungen hat,

durch Oxydation mit m-Chlorperbenzoesäure hergestellt, wobei die 4-(Chlor)-verbindungen in 44%-iger und die 4-(Toluolsulfonyloxy)-verbindungen [4-(Tosyl)-verbindungen] in 55%-iger Ausbeute anfallen. Außer den niedrigen Ausbeuten haben die bekannten Verfahren noch weitere Nachteile: Es muß in großen Volumina gearbeitet werden und das Oxydationsmittel ist eine schwer zugängliche, sich leicht zersetzende Verbindung.

Der Erfindung liegt die Aufgabe zugrunde, unter Behebung der Nachteile des Standes der Technik neue Pyrimidinderivat-Zwischenprodukte zur Herstellung von 6-(Amino)-1,2-di-(hydro)-1-(hydroxy)-2-(imino)-4-(piperidino)-pyrimidin [Minoxidil], durch welche dieses einfacher und leichter, insbesondere unter Verwendung von einfachen Reagenzien und unter gemäßigten Reaktionsbedingungen und damit ohne Nebenreaktionen, in überlegenen Ausbeuten und überlegener Reinheit hergestellt werden können, sowie ein Verfahren zur Herstellung dieser Pyrimidinderivat-Zwischenprodukte und von bekannten Pyrimidinderivaten, durch welches diese aus leicht zugänglichen, einfachen Ausgangssubstanzen in hohen Ausbeuten erhalten werden können, zu schaffen.

Das Obige wurde überraschenderweise durch die Erfindung erreicht.

Es wurde nämlich überraschenderweise festgestellt, daß die Nachteile der bekannten Verfahren nicht eintreten, wenn die 2,6-Di-(amino)-pyrimidinderivate der allgemeinen Formel II in Gegenwart von Wasserstoffperoxyd statt der m-Chlorperbenzoesäure mit Säureanhydriden der allgemeinen Formel III umgesetzt werden, wobei außer den Pyrimidinderivaten der allgemeinen Formel Id oder statt derer noch weitere Pyrimidinderivate, welche die nachfolgend angegebenen allgemeinen Formeln Ia, Ib beziehungsweise Ic haben, erhalten werden können, aus denen das als Arzneimittelwirkstoff verwendbare 6-(Amino)-1,2-di-(hydro)-1-(hydroxy)-2-(imino)-4-(piperidino)-pyrimidin wesentlich vorteilhafter als nach den bekannten Synthesen hergestellt werden kann.

Gegenstand der Erfindung sind daher Pyrimidinderivate der allgemeinen Formel(n)

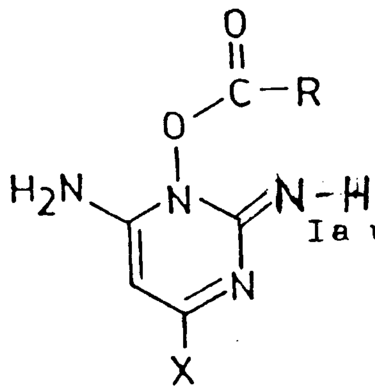

Ia und/oder

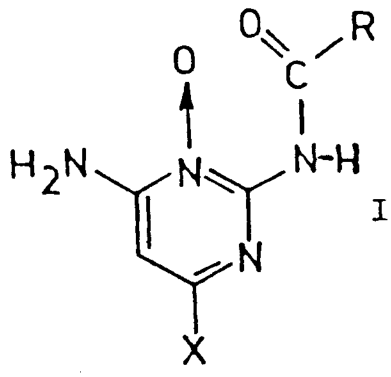

Ib und/oder

und/oder

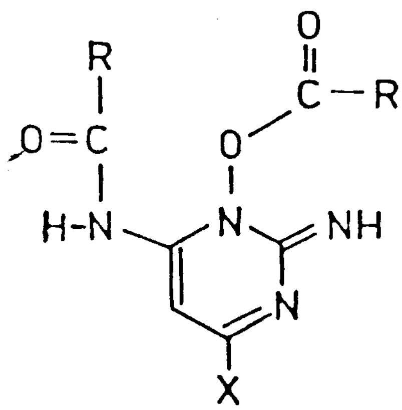

Ic,

worin

R für einen Alkylrest mit 1 bis 6 Kohlenstoffatom(en) oder einen, gegebenenfalls durch 1 oder mehr Halogenatom(e) substituierten, Arylrest mit 6 bis 12 Kohlenstoffatomen steht und

X Chlor oder Brom oder einen, gegebenenfalls 1-bis 3-fach substituierten, Arylsulfonyloxyrest mit einem Arylteil mit 6 - 12 Kohlenstoffatomen bedeutet.

Der Alkylrest, für den R stehen kann, kann jeder beliebige geradkettige oder verzweigte gesättigte Kohlenwasserstoffrest mit 1 bis 6 Kohlenstoffatom(en), zum Beispiel ein Methyl, Äthyl-, n- beziehungsweise Isopropyl-, n-, sek.- beziehungsweise tert.-Butyl-, n- beziehungsweise Isopentyl- beziehungsweise n- und Isohexylrest, sein.

Vorzugsweise ist der Alkylrest, für den R stehen kann, ein solcher mit 1 bis 4, insbesondere 1 oder 2, ganz besonders 1, Kohlenstoffatom(en).

Es ist auch bevorzugt, daß der Arylrest, für den R stehen kann, ein Phenyl- oder Naphthylrest, ganz besonders ein Phenylrest, ist.

Vorzugsweise ist der substituierte Arylrest, für den R stehen kann, durch 1 oder 2 Substituent(en) substituiert.

Ferner ist es besonders bevorzugt, daß das beziehungsweise die Halogenatom(e), durch welche[s] der Arylrest, für den R stehen kann, substituiert sein kann, [ein] Chlor- und/oder Bromatom(e), insbesondere Chloratom(e), ist beziehungsweise sind.

Weiterhin ist es bevorzugt, daß der Arylsulfonyloxyrest, für den X stehen kann, ein Phenylsulfonyloxy- oder Naphthylsulfonyloxyrest, ganz besonders ein Phenylsulfonyloxyrest, ist.

Besonders bevorzugt ist es, daß der beziehungsweise die Substituient(en) am Arylring des Arylsulfonyloxyrestes, für den X stehen kann, [ein] Alkylrest(e) mit 1 bis 4, vor allem 1 bis 3, insbesondere 1 oder 2, ganz besonders 1, Kohlenstoffatom(en) ist beziehungsweise sind.

Ganz besonders bevorzugt ist der Arylsulfonyloxyrest, für den X stehen kann, ein Toluolsulfonyloxyrest (Tosyloxyrest), vor allem p-Toluolsulfonyloxyrest, oder Mesitylensulfonyloxyrest.

Am meisten bevorzugt steht X für ein Chloratom.

Gegenstand der Erfindung ist auch ein Verfahren zur Herstellung der erfindungsgemäßen Verbindungen, und/oder von Pyrimidinderivaten der allgemeinen Formel

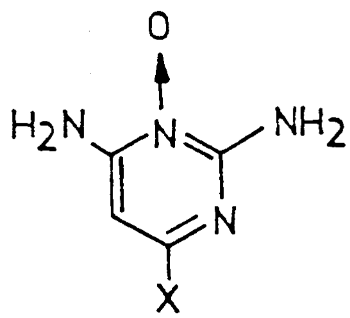

Id ,

worin

X die oben angegebenen Bedeutungen hat,

durch Umsetzung von 2,6-Di-(amino)-pyrimidinderivaten der allgemeinen Formel

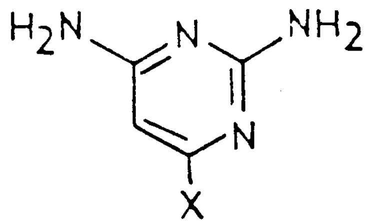

II ,

worin

X die oben angegebenen Bedeutungen hat,

mit einer Percarbonsäure, welches dadurch gekennzeichnet ist, daß die Umsetzung mit Säureanhydriden der allgemeinen Formel

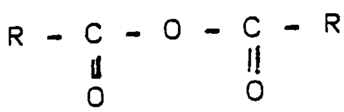

III ,

worin

R die oben angegebenen Bedeutungen hat,

in Gegenwart von Wasser und Wasserstoffperoxyd durchgeführt wird und das erhaltene Produktgemisch oder die reinen Pyrimidinderivate der allgemeinen Formel Ia, Ib, Ic beziehungsweise Id abgetrennt werden und gegebenenfalls die reinen Pyrimidinderivate der allgemeinen Formel Ia, Ib beziehungsweise Ic beziehungsweise Gemische derselben hydrolysiert werden und/oder gegebenenfalls die Pyrimidinderivate der allgemeinen Formel Id mit Säureanhydriden der allgemeinen Formel III,

worin

R die oben angegebenen Bedeutungen hat,

umgesetzt werden.

Die erfindungsgemäß herstellbaren Pyrimidinderivate der allgemeinen Formel Id können auch in tautomerer Form, in welcher in der 1 Stellung des Pyrimidinringes statt des Oxydsauerstoffes eine Hydrogruppe und statt der Aminogruppe in der 2-Stellung des Primidinringes eine Iminogruppe vorliegt sowie die Doppelbindung zwischen den Stellungen 1 und 2 des Pyrimidinringes in die Bindung zwischen dem Kohlenstoffatom der 2-Stellung des Pyrimidinringes und dem an ihn gebundenen Iminostickstoffatom umgeklappt ist, existieren. Auch auf diese richtet sich die Erfindung.

Die im erfindungsgemäßen Verfahren gegebenenfalls an den Pyrimidinderivaten der allgemeinen Formel(n) Ia, Ib und/oder Ic vorgenommene Hydrolyse liefert Pyrimidinderivate der allgemeinen Formel Id.

Im erfindungsgemäßen Verfahren werden die Ausgangsverbindungen der allgemeinen Formel II bevorzugt in einem hinsichtlich der Oxydationsreaktion inerten Lösungsmitteln, wie Alkoholen, Äthern, Estern und/oder Ketonen, zusammen mit dem notwendigen Volumen des unterschiedliche Wassermengen enthaltenden Wasserstoffperoxyds aufgelöst, und bei Temperaturen zwischen 40 °C und 90 °C wird zu der Lösung das entsprechende Säureanhydrid der Formel III gegeben. Aus den beiden Komponenten entsteht während der Reaktion eine Percarbonsäure, die die Ausgangsverbindung der allgemeinen Formel II oxydiert. Die Oxydation führt zu den N-Oxy-Derivaten der allgemeinen Formeln Ia bis Id.

Die Verbindungen der allgemeinen Formeln Ia und Ib sind Monoacylderivate, die sich darin unterscheiden, daß im Falle von Ia die Acylgruppe an das sich an das Stickstoffatom in 1-Stellung anschließenden Sauerstoffatom gebunden ist, während im Falle von Ib die Acylgruppe an der Aminogruppe in 2-Stellung gebunden vorliegt. Das N-Acylderivat der allgemeinen Formel Ib geht unter außerordentlich schonenden Bedingungen, schon bei Auflösen oder schwachem Erwärmen oder durch Einwirkung von Säure-, Basen- oder Wasserspuren in das O-Acylderivat der allgemeinen Formel Ia über. Das N-Oxyd-Derivat der allgemeinen Formel Ic ist eine Diacylverbindung, in der die eine Acylgruppe an das sich an das Stickstoffatom in 1-Stellung anschließende Sauerstoffatom gebunden ist und die andere an der Aminogruppe in 6-Stellung hängt. Wie bereits erwähnt, sind die N-Oxyd-Derivate der allgemeinen Formel Id bekannt; sie entstehen leicht aus den N-Oxy-Derivaten der allgemeinen Formeln Ia, Ib und Ic durch schwach alkalische Einwirkung. Umgekehrt bilden sich aus den Verbindungen der allgemeinen Formel Id durch eine schonende Säureanhydridbehandlung Verbindungen der allgemeinen Formeln Ia bis Ic.

Bei der praktischen Ausführung des erfindungsgemäßen Verfahrens hängt das Verhältnis der Produkte Ia bis Id von den Verfahrensbedingungen ab. In erster Linie wird das Produktverhältnis von der Art der Aufarbeitung des Reaktionsgemisches, der Art des Lösungsmittels und dem Molverhältnis der Reagenzien beeinflußt.

Wird das Reaktionsgemisch nach der Umsetzung durch Behandeln mit Wasser und Lauge aufgearbeitet, so gehen die primär gebildeten Acylverbindungen Ia bis Ic quantitativ in die Verbindung Id über, und nur diese ist isolierbar. Wird eine derartige Behandlung bei der Aufarbeitung nicht vorgenommen, so können die gebildeten Verbindungen Ia bis Ic isoliert werdend. Möglich ist es auch, zunächst den auskristallisierten Teil der Verbindungen Ia bis Ic abzutrennen, dann die Mutterlauge alkalisch zu machen und die entstandene Verbindung der allgemeinen Formel Id zu isolieren.

Das erfindungsgemäße Verfahren zeigt eine eigenartige Abhängigkeit vom Lösungsmittel. Die als Ausgangsstoffe eingesetzten 2,6-Di-(amino)-pyrimidinderivate der allgemeinen Formel II lösen sich in den meisten organischen Lösungsmitteln außerordentlich schlecht. Überraschenderweise wurde jedoch gefunden, daß sie in Gegenwart einer geringen Menge Wasser, insbesondere auf 1 Mol der Verbindung II gerechnet 2-20 Mol, unter leichtem Erwärmen in den meisten organischen Lösungsmitteln in Lösung gehen und dadurch ihre Umsetzung möglich wird. Nach einer zweckmäßigen Ausführungsform des erfindungsgemäßen Verfahrens werden daher je Mol 2,6-Di-(amino)-pyrimidinderivat der allgemeinen Formel II 2 bis 20 Mol Wasser eingesetzt. Bei der praktischen Ausführung des Verfahrens wird die zum Auflösen erforderliche Menge Wasser bevorzugt mit dem Wasserstoffperoxyd zusammen zu dem Gemisch gegeben. Unter sonst gleichen Bedingungen hängt das Produktverhältnis stark vom eingesetzten Lösungsmittel ab. Im Fall, daß X für Chlor oder Brom steht, scheidet sich beispielsweise in Äthanol ein Gemisch der Verbindungen Ia und Ib aus, während in Tetrahydrofuran praktisch reines O-Acylderivat der allgemeinen Formel Ia entsteht. In tert.-Butanol oder Äthylacetat wird, begleitet von wenig Ic, hauptsächlich Ia gebildet. Steht X für eine Tosylgruppe, so bildet sich in Äthanol die Verbindung Ia, und die Verbindung Ib kann überhaupt nicht isoliert werden.

Verwendet man ein und dasselbe Lösungsmittel und erhöht die Menge des Säureanhydrids, so verschiebt sich das Verhältnis in Richtung des Diacylderivates der allgemeinen Formel Ic, überraschenderweise selbst dann, wenn man die Umsetzung in wässrigem Medium vornimmt. Die Art des Substituenten R hat auf das Produktverhältnis keinen Einfluß.

Es wurde bereits erwähnt, daß die zur Herstellung der Verbindungen der allgemeinen Formel Id beziehungsweise sonstiger, analoger Verbindungen bekannten Verfahren niedrige Ausbeuten ergeben, zum Arbeiten mit großen Volumina zwingen und ein sich leicht zersetzendes Oxydationsmittel erfordern. Das erfindungsgemäße Verfahren hat diese Nachteile nicht. Die Ausbeuten sind höher, das Oxydationsmittel bildet sich aus einfachen Chemikalien während der Reaktion und die zu handhabenden Volumina sind wesentlich kleiner als im Falle der bekannten Verfahren. Ein möglicher Grund für die höhere Ausbeute kann darin gesehen werden, daß das Oxydationsmittel auch am Aufbau der Schutzgruppen (Acylgruppen) der empfindlichen Substituenten der herzustellenden Pyrimidinderivate der allgemeinen Formel(n) Ia und/oder Ib und/oder Ic beteiligt ist. Dadurch ist das Molekül vor Überoxydation geschützt.

Die im erfindungsgemäßen Verfahren als Ausgangssubstanzen dienenden 2,6-Di-(amino)-pyrimidinderivate der allgemeinen Formel II sind bekannt. Die als Substituenten X Chlor oder Brom aufweisenden 2,6-Di-(amino)-pyrimidinderivate der allgemeinen Formel II sind in der US-PS 3 644 364 beschrieben, während die

als Substituenten X einen Arylsulfonyloxyrest aufweisenden 2,6-Di-(amino)-pyrimidinderivate der allgemeinen Formel II aus der HU-Patentschrift 177 601 bekannt sind.

Durch die erfindungsgemäßen Zwischenprodukte Pyrimidinderivate der allgemeinen Formel(n) Ia und/oder Ib und/oder Ic wird ein neuer Weg durch ein chemisch eigenartiges Verfahren zur Herstellung des therapeutisch wertvollen, nämlich den Blutdruck senkenden und den Haarwuchs fördernden, Endproduktes 6-(Amino)-1,2-di-(hydro)-1-(hydroxy)-2-(imino)-4-(piperidino)-pyrimidin [Minoxidil] der allgemeinen Formel

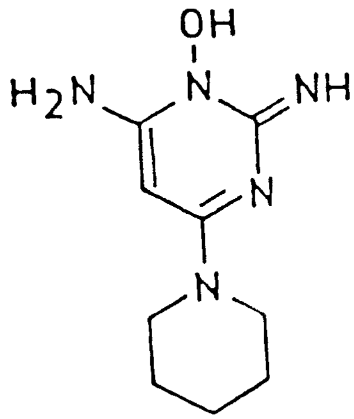

V ,

der technisch fortschrittlich ist, eröffnet.

Die Weiterumsetzung der erfindungsgemäßen Pyrimidinderivate der allgemeinen Formel(n) Ia und/oder Ib und/oder Ic zum 6-(Amino)-1,2-di-(hydro)-1-(hydroxy)-2-(imino)-4-(piperidino)-pyrimidin der Formel V kann durch Umsetzen der ersteren mit Piperidin und Hydrolyse der erhaltenen [eine Acyl- beziehungsweise Acyloxygruppe(n) aufweisenden 4-(Piperidino)-pyrimidinderivate der allgemeinen Formel(n)
meinen Formel(n)

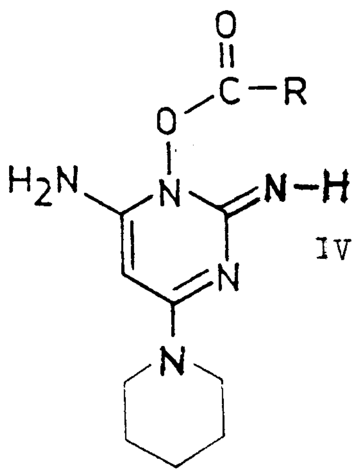

IVa und/oder

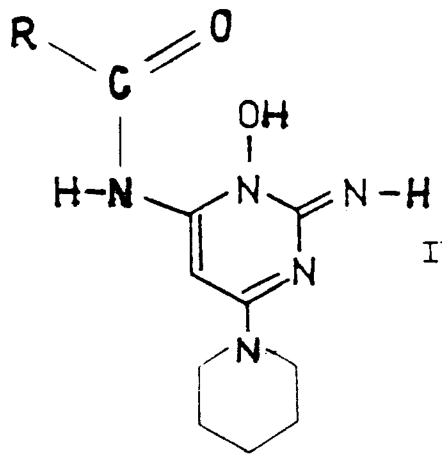

IVb und/oder

und/oder

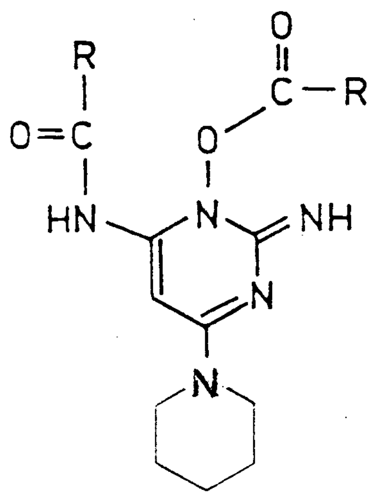

IVc ,

worin

R die obigen Bedeutungen hat,

gegebenenfalls nach ihrem Isolieren, durchgeführt werden. Dieses Verfahren ist Gegenstand der HU-A-196 067. Durch es kann das 6-(Amino)-1,2-di-(hydro)-1-(hydroxy)-2-(imino)-4-(piperidino) -pyrimidin einfacher und in wesentlich höherer Ausbeute als nach den bekannten Verfahren hergestellt werden. Während das beste der bekannten Verfahren (das gemäß der ungarischen Patentschrift 177 601) eine Gesamtausbeute von etwa 20 bis 21% liefert, kann über das erfindungsgemäße Verfahren und die erfindungsgemäßen Zwischenprodukte Pyrimidinderivate der allgemeinen Formel(n) Ia und/oder Ib und/oder Ic das 6-(Amino)-1,2-di-(hydro)-1-(hydroxy)-2-(imino)-4-(piperidino)-pyrimidin in einer Ausbeute von 49 bis 50%, bezogen auf die Ausgangsstoffe 2,6-Di-(amino)-pyrimidinderivateder allgemeinen Formel II hergestellt werden. Das bedeutet im Vergleich zum Stand der Technik eine sehr bedeutende und nicht vorhersehbare Verbesserung.

Die Umsetzung der Pyrimidinderivate der allgemeinen Formel(n) Ia und/oder Ib und/oder Ic mit dem Piperidin wird zweckmäßig bei Temperaturen von 0 bis 100°C, vorzugsweise Raumtemperatur, durchgeführt. Es ist auch zweckmäßig, sie in einem Lösungsmittel vorzunehmen. Die Wahl der Reaktionstemperatur hängt von den übrigen Reaktionsbedingungen, wie vom Lösungsmittel, ab. Die Reaktionszeit beträgt im allgemeinen 5 Minuten bis einige Stunden. Im allgemeinen sind auch bei Raumtemperatur höchstens 2 Stunden für den Ablauf der Reaktion notwendig, während mit einer Erhöhung der Temperatur die Reaktionszeit auch im Vergleich hierzu bedeutend verkürzt werden kann. Bei der Umsetzung kann das Piperidin selbst als Lösungsmittel dienen, es können aber auch andere Lösungsmittel verwendet werden. So kann die Umsetzung in protischen Lösungsmitteln, vorzugsweise Äthanol, in dipolar-aprotischen Lösungsmitteln, vorzugsweise Acetonitril, oder in einem apolar-aprotischen Medium, vorzugsweise Chloroform, durchgeführt werden. Unter solchen Bedingungen werden die [eine] Acylbeziehungsweise Acyloxygruppe(n) aufweisenden 4-(Piperidino)-pyrimidinderivate der allgemeinen Formel(n) IVa und/oder IVb und/oder IVc

praktisch quantitativ erhalten.

Die Hydrolyse der erhaltenen [eine] Acyl- beziehungsweise Acylogygruppe(n) aufweisenden 4-(Piperidino)-pyrimidinderivate der allgemeinen Formel(n) IVa und/oder IVb und/oder IV c läuft, wenn das Piperidin auch die Aufgabe des Lösungsmittels erfüllt, bereits bei Raumtemperatur schnell ab, so daß die [eine] Acyl- beziehungsweise Acyloxygruppe(n) aufweisenden 4-(Piperidino)-pyrimidinderivate der allgemeinen Formel(n) IVa und/oder IVb und/oder IVc nicht einmal isolierbar sind, oder sie geht innerhalb Minuten unter den Bedingungen der Aufarbeitung, auf Einwirkung von Wasser und einer Base, zum Beispiel von wäßriger Alkalilauge, vor sich. Das Verfahren kann auch so gelenkt werden, daß die Zwischenprodukte [eine] Acyl- beziehungsweise Acyloxygruppe(n) aufweisende 4-(Piperidino)-pyrimidinderivate der allgemeinen Formel(n) IVa und/oder IVb und/oder IVc in hoher Ausbeute und großer Reinheit isolierbar sind.

Das so hergestellte 6-(Amino)-1,2-di-(hydro)-1-(hydroxy)-2-(imino)-4-(piperidino)-pyrimidin der Formel V ist in kristalliner Form, in hoher Reinheit isolierbar und wird nicht von nachweisbaren Nebenprodukten begleitet.

Die Erfindung wird an Hand der folgenden Beispiele näher erläutert, wobei die Beispiele 1 bis 10 das erfindungsgemäße Verfahren und die Beispiele I bis IX die Weiterumsetzung der durch dieses erhaltenen Produkte betreffen.

Beispiel 1

2-Acetamido-4-chlor-6-amino-pyrimidin-1-oxyd und 6-Amino-1,2-dihydro-1-acetoxy-2-imino-4-chlor-pyrimidin

5,0 g (0,035 Mol) 2,6-Diamino-4-chlor-pyrimidin werden in 70 ml wasserfreiem Äthanol gelöst. Die Lösung wird unter Rühren, bei 40 °C innerhalb einer halben Stunde tropfenweise mit 7 ml 70 gew.-%-iger wäßriger Wasserstoffperoxydlösung und 14 ml Essigsäureanhydrid versetzt. Das Gemisch wird bei 60 °C weitere zwei Stunden lang gerührt. Nach dem Abkühlen werden die flockigen Kristalle abfiltriert, mit Äthanol gewaschen und dann getrocknet. Man erhält 2,67 g (38 %) 2-Acetamido-4-chlor-6-amino-pyrimidin-1-oxyd.
IR-Spektrum (KBr): 3400, 1690, 1640, 1610 $cm^{-1}$.

Die Mutterlauge wird im Vakuum eingedampft, der Rückstand mit 20 ml Wasser versetzt und das Gemisch über Nacht in den Kühlschrank gestellt. Die Kristalle werden abfiltriert, mit Wasser gewaschen und dann getrocknet. Man erhält 1,7 g (24 %) 6-Amino-1,2-dihydro-1-acetoxy-2-imino-4-chlor-pyrimidin.
IR-Spektrum (KBr): 3420, 1730, 1660, 1570, 1550 $cm^{-1}$
UV-Spektrum (EtOH): $\lambda_{max}$: 247, 276, 325 nm
NMR-Spektrum ($CDCl_3$ + TFA-d): 2,47 (s, 3H), 7,98 (s, 1H).

Beispiel 2

6-Amino-1,2-dihydro-1-acetoxy-2-imino-4-chlor-pyrimidin

5 g (0,035 Mol) 2,6-Diamino-4-chlor-pyrimidin werden in 50 ml wasserfreiem Tetrahydrofuran gelöst und die Lösung bei 40 °C innerhalb einer halben Stunde tropfenweise mit 7 ml 70 gew.-%-iger wässriger Wasserstoffperoxydlösung und 16 ml Essigsäureanhydrid versetzt. Das Gemisch wird bei 60 °Cweitere 2 Stunden gerührt. Nach dem Abdampfen des Tetrahydrofurans wird der Rückstand mit 50 ml Wasser versetzt und über Nacht im Kühlschrank stehengelassen. Man erhält 4,00 g (57 %) der Titelverbindung. Durch Abtrennen der zweiten Kristallfraktion erhält man weitere 0,77 g (11 %) der Verbindung. Das IR-, UV- und NMR-Spektrum des Produktes stimmt mit den im Beispiel 1 angegebenen Daten überein.

Beispiel 3

6-Acetamido-1,2-dihydro-1-acetoxy-2-imino-4-chlor-pyrimidin und 6-Amino-1,2-dihydro-1-acetoxy-2-imino-4-chlor-pyrimidin

In 170 ml tert.-Butanol werden bei 50 °C 17,28 g (0,12 Mol) 2,6-Diamino-4-chlor-pyrimidin eingerührt und dann zu dem Gemisch 17 ml 30 gew.-%-ige wäßrige Wasserstoffperoxydlösung gegeben. Zu der erhaltenen Lösung werden bei 55-60 °C innerhalb einer Stunde 36 ml Essigsäureanhydrid tropfenweise zugegeben. Das Gemisch wird eine weitere Stunde lang bei der angegebenen Temperatur gerührt, dann auf 15 °C gekühlt und 2 Stunden lang stehengelassen. Die ausgefallenen Kristalle werden abfiltriert, zweimal mit je 20 ml Wasser, dann zweimal mit je 20 ml Äthanol gewaschen und schließlich getrocknet. Auf diese Weise erhält man 15 g (62 %) eines Produktes, das zu 30 % aus der Diacetyl- und zu 70 % aus der

Monoacetylverbindung gemäß Titel besteht. Zu der Mutterlauge werden die Lösung von 6 g Natriumpyrosulfit in 12 ml Wasser und dann noch 170 ml Wasser gegeben. Das Gemisch wird bei Raumtemperatur eine halbe Stunde lang gerührt, dann im Vakuum auf das halbe Volumen eingedampft und mit wässriger Natronlauge auf pH 6 eingestellt. Das Gemisch wird über Nacht im Kühlschrank stehengelassen, das Produkt abfiltriert, dreimal mit je 20 ml Wasser gewaschen und dann getrocknet. Man erhält 2,4 g (10 %) der Monoacetylverbindung.

IR-, UV- und NMR-Spektrum der Monoacetylverbindung weisen die im Beispiel 1 angegebenen Daten auf.

IR-Spektrum der Diacetylverbindung (KBr): 1720, 1690, 1600, 1570 $cm^{-1}$.

Beispiel 4

6-Acetamido-1,2-dihydro-1-acetoxy-2-imino-4-chlor-pyrimidin

a) 6-Amino-1,2-dihydro-1-hydroxy-2-imino-4-chlor-pyrimidin (Verbindung der Formel Id [solche liegen in tautomeren Formen vor, indem die Doppelbindung umklappt und das Wasserstoffatom der Aminogruppe in der 2-Stellung unter Zurücklassen einer Iminogruppe an den Oxydsauerstoff in der 1-Stellung und unter Bildung einer Hydroxygruppe in der 1-Stellung geht])

Es werden 6,06 g (0,03 Mol) wie im Beispiel 1, 2 oder 3 beschrieben erhaltenes 6-Amino-1,2-dihydro-1-acetoxy-2-imino-4-chlor-pyrimidin in 18 ml (0,036 Mol) einer wäßrigen 2 n Natronlauge gelöst. Die Lösung wird 1 Stunde lang bei 60 bis 80°C gerührt, dann gekühlt und stehengelassen. Die ausgefallenen Kristalle werden abfiltriert, gewaschen und getrocknet. Schmelzpunkt: 193°C.

b) 6-Acetamido-1,2-dihydro-1-acetoxy-2-imino-4-chlorpyrimidin

Ein Gemisch aus 3,2 g (0,02 Mol) des wie vorstehend angegeben erhaltenen 6-Amino-1,2-dihydro-1-hydroxy-2-imino-4-chlor-pyrimidines und 30 ml Essigsäureanhydrid wird bei Raumtemperatur eine Stunde lang gerührt und dann mit 200 ml Äther versetzt. Die weißen Kristalle werden abfiltriert, mit Äther gewaschen und dann getrocknet. Man erhält 3,9 g (80 %) der Titelverbindung, deren physikalische Konstanten mit den im Beispiel 3 angegebenen übereinstimmen.

Beispiel 5

6-Amino-1,2-dihydro-1-acetoxy-2-imino-4-chlor-pyrimidin und 6-Acetamido-1,2-dihydro-1-acetoxy-2-imino-4-chlor-pyrimidin

4,32 g (0,03 Mol) 2,6-Diamino-4-chlor-pyrimidin und 9,3 ml 30 gew.-%-iges wäßriges Wasserstoffperoxyd werden bei 60 °C in 150 ml Wasser gelöst. Zu der Lösung werden bei 55-60 °Cunter Rühren innerhalb von 40 Minuten tropfenweise 18 ml Essigsäureanhydrid gegeben. Das Reaktionsgemisch wird bei der angegebenen Temperatur noch weitere 1½ Stunden lang gerührt, dann auf 15 °C abgekühlt und noch zwei Stunden der Niederschlag abfiltriert. Das Produkt wird mit Wasser gewaschen und dann getrocknet. Man erhält 1,45 g (20 %) 6-Acetamido-1,2-dihydro-1-acetoxy-2-amino-4-chlor-pyrimidin.

Die Mutterlauge wird mit 40 gew.-%-iger wäßriger Natronlauge auf pH 6 eingestellt und über Nacht im Kühlschrank stehengelassen. Die Kristalle werden abfiltriert, mit Wasser gewaschen und dann getrocknet. Man erhält 1,2 g (20 %) 6-Amino-1,2-dihydro-1-acetoxy-2-imino-4-chlor-pyrimidin. Die physikalischen Daten der Produkte stimmen mit den in den Beispielen 3 und 1 angegebenen Daten der entsprechenden Produkte überein.

Beispiel 6

6-Acetamido-1,2-dihydro-1-acetoxy-2-imino-4-p-toluol-sulfonyloxy-pyrimidin

84 g (0,3 Mol) 2,6-Diamino-4-tosyloxy-pyrimidin werden in 1200 ml wasserfreiem Tetrahydrofuran suspendiert. Zu der Suspension werden bei Raumtemperatur tropfenweise, unter Rühren 40 ml 70 gew.-%-iges wässriges Wasserstoffperoxyd gegeben, wodurch eine klare Lösung entsteht. Zu dieser werden unter Rühren bei 40 °C innerhalb einer Stunde 200 ml Essigsäureanhydrid gegeben. Das Gemisch wird anschließend noch zwei Stunden lang bei 60 °C gerührt, dann das Lösungsmittel in Vakuum entfernt und der Rückstand über Nacht in den Kühlschrank gestellt. Die ausgefallene Substanz wird abfiltriert, mit Tetrahydrofuran gewaschen und dann getrocknet. Man erhält 77 g (68 %) der Titelverbindung, die bei 196-200 °C schmilzt.

IR-Spektrum (KBr): 1720, 1690, 1600, 1580, 1500 $cm^{-1}$.
UV-Spektrum (EtOH): $\lambda_{max}$ 252, 285, 321 nm.
NMR-Spektrum ($CDCl_3$ + TFA-d): 2,38 (s, 3H), 2,48 (s, 3H), 2,55 (s, 3H), 7,42 (s, 1H), 7,46 und 7,98 (dd, 4H).

Beispiel 7

6-Amino-1,2-dihydro-1-acetoxy-2-imino-4-p-toluol-sulfonyloxy-pyrimidin

2,8 g (0,001 Mol) 2,6-Diamino-4-tosyloxy-pyrimidin werden in 70 ml wasserfreiem Äthanol suspendiert. Zu der Suspension werden bei 40 °C tropfenweise, unter Rühren 5 ml 70 gew.-%-iges wässriges Wasserstoffperoxyd und danach 3 ml Essigsäureanhydrid gegeben. Bei Erwärmen auf 60 °Cerhält man eine klare Lösung, die beim Stehen langsam opal wird. Dünnschichtchromatographisch wird festgestellt, daß die Reaktion ebgelaufen ist und neben der Titelverbindung auch etwas Diacetylverbindung entstanden ist. Durch Filtrieren werden 0,35 g einer Festsubstanz abgetrennt. Wird das Äthanol verdampft und der Rückstand in dem Kühlschrank gestellt, so erfolgt keine Abscheidung. Nach Dekantieren des Wassers und Aufnehmen des Rückstandes in Äthanol erhält man ein kristallines Produkt. Ausbeute: 1,95 g (54 %) der Titelverbindung.
IR-Spektrum (KBr): 3440, 1720, 1560, 1660, 1600 $cm^{-1}$
UV-Spektrum (EtOH) $\lambda_{max}$: 244, 260 sh, 322 nm
NMR-Spektrum ($CDCl_3$ + TFA-d) $\delta$: 2,44 (s, 3H), 3,50 (s, 3H), 7,53 (s, 1H), 7,48 und 8,00 (dd, 4H).

Beispiel 8

6-Amino-1,2-dihydro-1-propionoxy-2-imino-4-chlor-pyrimidin

8,54 g (0,06 Mol) 2,6-Diamino-4-chlor-pyrimidin werden bei 40 °C in einem Gemisch aus 80 ml tert.-Butanol und 5,3 ml 70 gew.-%-igem wäßrigem Wasserstoffperoxyd gelöst.Die Lösung wird auf 60 °C erwärmt und unter Rühren tropfenweise mit 18 ml Propionsäureanhydrid versetzt, wobei durch Regulieren der Temperatur des äußeren Bades dafür zu sorgen ist, daß die Temperatur des Reaktionsgemisches 60 ± 2 °Cbeträgt. Nach Beendigung der Zugabe wird das Reaktionsgemisch zwei Stunden lang bei der angegebenen Temperatur gerührt und dann auf Raumtemperatur gekühlt. Nach zwei Stunden wird das ausgefallene Produkt abfiltriert, mit kaltem Äthanol gewaschen und dann getrocknet. Man erhält 6,34 g (49 %) der Titelverbindung. Aus der Mutterlauge scheiden sich über Nacht weitere 0,6 g (4 %) Produkt ab.
IR-Spektrum (KBr): 1760 $cm^{-1}$ (C=O)
$^1$H-NMR-Spektrum (DMSO-$d_6$) $\delta$: 1,16 (t, 3H, $CH_3$), 2,65 (q, 2H, $CH_2$), 7,60 (s, 1H, $C_5$-H), 7,62 (breit, 1H, HN=).

Beispiel 9

6-Amino-1,2-dihydro-1-acetoxy-2-imino-4-chlor-pyrimidin und 6-Acetamido-1,2-dihydro-1-acetoxy-2-imino-4-chlor-pyrimidin

In einem Gemisch aus 43 ml Äthylacetat und 3 ml 70 gew.-%-igem wässrigem Wasserstoffperoxyd werden bei 50 °C 4,32 g (0,03 Mol) 2,6-Diamino-4-chlor-pyrimidin gelöst. Zu der Lösung werden bei 55-60 °C innerhalb von 90 Minuten 12 ml Essigsäureanhydrid gegeben. Das Gemisch wird bei der genannten Temperatur noch eine halbe Stunde lang gerührt, dann abgekühlt und über Nacht in den Kühlschrank gestellt. Die ausgefallenen Kristalle werden abfiltriert, mit Äthylacetat gewaschen und dann getrocknet. Man erhält 1,80 g (25 %) 6-Acetamido-1,2-dihydro-1-acetoxy-2-imino-4-chlor-pyrimidin.

Die Mutterlauge wird mit 40 ml 10 gew.-%-iger wäßriger Natronlauge und zweimal mit 40 ml Wasser extrahiert. Dann wird die organische Phase im Vakuum auf die Hälfte ihres Volumens eingedampft, der Niederschlag wird abfiltriert und mit Wasser gewaschen. Man erhält 1,25 g (20 %) 6-Amino-1,2-dihydro-1-acetoxy-2-imino-4-chlor-pyrimidin. Aus der vereinigten wässrig-alkalischen Phase fallen über Nacht weitere 0,6 g (10 %) des Produktes aus.

Beispiel 10

6-Acetamido-1-acetoxy-2-imino-4-mesitylen-sulfonyloxy-1,2-dihydro-pyrimidin

In einem mit Rührer, Rückflußkühler und Tropftrichter ausgerüsteten Rundkolben von 1000 ml Volumen werden 45,8 g (0,15 Mol) 2,4-Diamino-6-mesitylen-sulfonyloxy-pyrimidin in 600 ml Tetrahydrofuran suspendiert. Zu der Suspension werden unter Rühren 20 ml (27,2 g) 70 gew.-%-iges wäßriges Wasserstoffparoxyd gegeben, wobei die Substanz in Lösung geht. Die Lösung wird auf dem Ölbad allmählich erwärmt. Wenn die Temperatur 40 °C erreicht hat, werden tropfenweise 100 ml (108,1 g, 1,06 Mol) Essigsäureanhydrid zugesetzt, wobei darauf zu achten ist, daß die Temperatur 60 °C nicht übersteigt. Nach Beendigung der Zugabe wird des Gemisch bei 60 °C noch zwei Stunden lang gerührt. Dann wird auf dem 60 °C warmen Bad das Tetrahydrofuran im Vakuum entfernt und der Rückstand in den Kühlschrank gestellt. Die ausgefallene Substanz wird abfiltriert und mit Tetrahydrofuran gewaschen. Man erhält 34,6 g (56,5 %) der Titelverbindung in Form einer weißen, kristallinen Substanz, die bei 162-163 °C unter Zersetzung schmilzt. Das Produkt ist chromatographisch einheitlich.

| Elementaranalyse für $C_{17}H_{20}N_4O_8S$ | | | | |
|---|---|---|---|---|
| berechnet, %: | C 49,99 | H 4,94 | N 13,72 | S 7,85 |
| gefunden, %: | C 49,94 | H 4,94 | N 13,95 | S 7,76 |

IR-Spektrum (KBr): 3400, 1720, 1695, 1580, 1200, 1175, 1050 $cm^{-1}$
NMR-Spektrum (DMF) δ: 2,10, 2,25, 2,35 (3H, s), 2,6 (6H, s, 2',6'-$CH_3$), 6,6 (1H, s, 5-H)x, 6,85 (2H, s, 3',5'-H).

Beispiel I

Herstellung von 6-Amino-1,2-dihydro-1-hydroxy-2-imino-4-piperidino-pyrimidin

Zum Gemisch von 10 ml Äthanol und 3 ml Piperidin werden unter Rühren 1,01 g (5 mMol) 6-Amino-1,2-dihydro-1-acetoxy-2-imino-4-chlorpyrimidin gegeben. Das Gemisch wird unter Rühren eine halbe Stunde lang gekocht. Dann werden 5 ml wässrige n Natriumhydroxydlösung zugegeben, und es wird eine weitere halbe Stunde lang gekocht. Das Gemisch wird im Vakuum eingedampft und der Rückstand mit 10 ml Wasser vermischt. Die erhaltenen Kristalle werden abfiltriert, mit Wasser gewaschen und getrocknet.
So erhält man 0,85 g (82 %) der Titelverbindung.
Schmelzpunkt: 262 - 266 °C.
IR-Spektrum ($cm^{-1}$): 3450, 3420, 3400, 3370, 3260, 1655, 1250, 1210, 1165, 1020
$^1$H-NMR-Spektrum (DMSO-$d_6$): 1,52; 3,40; 5,36; 6,84
$^{13}$C-NMR-Spektrum (DMSO-$d_6$ + $CD_3OD$): 156,6; 153,7; 152,1; 74,1; 45,7; 25,7; 24,7.

Beispiel II

Herstellung Von 6-Amino-1,2-dihydro-1-hydroxy-2-imino-4-piperidino-pyrimidin

Unter Rühren werden 2,02 g (10 mMol) 6-Amino-1,2-dihydro-1-acetoxy-2-imino-4-chlorpyrimidin bei Raumtemperatur zu 8 ml Piperidin gegeben. Das Gemisch wird bei Raumtemperatur zwei Stunden lang gerührt, dann wird das Piperidin im Vakuum abdestilliert. Der Rückstand wird im Gemisch von 20 ml Äthanol und 10 ml wäßriger n Natriumhydroxydlösung aufgenommen und eine halbe Stunde lang am Rückfluss gekocht, danach im Vakuum eingedampft. Der Rückstand wird in 20 ml Wasser aufgenommen, die Kristalle werden abfiltriert, mit Wasser gewaschen und getrocknet. So erhält man 1,74 g (86 %) der Titelverbindung, die bei Mischen mit dem Produkt gemäss Beispiel I keine Schmelzpunktdepression ergibt.

Beispiel III

Herstellung von 6-Amino-1,2-dihydro-1-hydroxy-2-imino-4-piperidino-pyrimidin

Zum Gemisch von 10 ml Chloroform und 2 ml Piperidin werden unter Rühren 0,3 g (0,88 mMol) 6-Amino-1,2-dihydro-1-acetoxy-2-imino-4-p-toluol-sulfonyloxy-pyrimidin gegeben. Das Gemisch wird unter Rühren eine halbe Stunde lang gekocht und im Vakuum eingedampft. Es werden 5 ml Äthanol und 1 ml wäßrige n Natriumhydroxydlösung zugegeben. Dann lässt man das Gemisch bei Raumtemperatur eine Stunde lang stehen, danach es im Vakuum eingedampft. Der Rückstand wird mit 10 ml Wasser vermischt, die Kristalle werden abfiltriert, mit Wasser gewaschen und getrocknet.

So erhält man 0,14 g (75 %) der Titelverbindung, die bei Vermischen mit dem Produkt gemäss Beispiel I keine Schmelzpunktdepression ergibt.

Beispiel IV

Herstellung von 6-Amino-1,2-dihydro-1-hydroxy-2-imino-4-piperidino-pyrimidin

Zum Gemisch von 10 ml Chloroform und 2 ml Piperidin werden unter Rühren 0,49 g (2 mMol) 6-Acetamido-1,2-dihydro-1-acetoxy-2-imino-4-chlorpyrimidin gegeben. Dann wird das Gemisch eine halbe Stunde lang am Rückfluss gekocht und im Vakuum eingedampft. Der Rückstand wird im Gemisch von 10 ml Athanol und 3 ml wäßriger n Natriumhydroxydlösung aufgelöst, dann bei Raumtemperatur eine Stunde lang stehengelassen und erneut im Vakuum eingedampft. Der Rückstand wird in 10 ml Wasser aufgenommen, die Kristalle werden abfiltriert, mit Wasser gewaschen und getrocknet. So erhält man 0,34 g (80 %) der Titelverbindung, die bei Vermischen mit dem Produkt gemäss Beispiel I keine Schmelzpunktdepression ergibt.

Beispiel V

Herstellung von 6-Amino-1,2-dihydro-1-hydroxy-2-imino-4-piperidino-pyrimidin

Zu 760 ml wasserfreiem Piperidin werden bei einer Temperatur von 0 - 5 °C unter Rühren 76 g (0,2 Mol) 6-Acetamido-1,2-dihydro-1-acetoxy-2-imino-4-p-toluol-sulfonyloxypyrimidin gegeben. Das Rühren wird bei dieser Temperatur fortgesetzt, nach zwei Stunden lässt man die Temperatur des Gemisches sich auf Raumtemperatur erhöhen, und es wird 24 Stunden lang gerührt. Das Piperidin wird im Vakuum abdestilliert, und zum Rückstand werden 500 ml Wasser gegeben. Das Gemisch wird bis zum nächsten Tag in den Kühlschrank gestellt. Der abgesonderte Stoff wird abfiltriert, mit Wasser gewaschen und gründlich abgesaugt. Der auf dem Filter verbliebene Stoff wird dreimal mit 50 ml Äther suspendiert, gewaschen und dann getrocknet. So erhält man 23,0 g (55 %) der Titelverbindung.

Zur Mutterlauge werden 75 ml 10 gew.-%-ige Natriumhydroxydlösung gegeben, dann wird eingedampft. Zum Rückstand gibt man 200 ml Wasser, und der pH der Lösung wird auf 7 eingestellt. Die Lösung wird bis zum nächsten Tag im Kühlschrank gelassen, dann wird der auskristallisierte Stoff abfiltriert, mit Wasser gewaschen und getrocknet. Man erhält weitere 8,9 g (21 %) der Titelverbindung.
So erhält man insgesamt 31,9 g (76 %) der Titelverbindung, die bei Vermischen mit dem Produkt gemäss Beispiel I keine Schmelzpunktdepression ergibt.

Beispiel VI

Herstellung von 6-Amino-1,2-dihydro-1-hydroxy-2-imino-4-piperidino-pyrimidin

a) Herstellung von 6-Acetamido-1,2-dihydro-1-hydroxy-2-imino-4-piperidino-pyrimidin

Zum Gemisch von 20 ml Chloroform und 5 ml Piperidin werden 1,01 g (5 mMol) 6-Amino-2-acetamido-4-chlorpyrimidin-1-oxyd gegeben. Das Gemisch wird unter Rühren eine halbe Stunde lang gekocht. Die erhaltene Lösung wird gekühlt und dreimal mit 10 ml n Salzsäure extrahiert, dann dreimal mit 10 ml Wasser gewaschen. Die Chloroform-Phase wird über ausgeglühtem Natriumsulfat getrocknet und im Vakuum eingedampft. Der Rückstand wird mit 50 ml Äther verrieben, die Kristalle werden abfiltriert, mit Äther gewaschen und getrocknet.
So erhält man 0,86 g (69 %) der Titelverbindung.
Schmelzpunkt: 204 - 205 °C.
IR-Spektrum (KBr): 1670, 1600, 1570, 1500 $cm^{-1}$
UV-Spektrum (EtOH): 245, 325 nm
NMR-Spektrum ($CDCl_3$ + $CD_3OD$): 1,63 (m, 6H), 2,30 (s, 3H), 3,57 (m, 6H), 7,04 (s, 1H).

b) Herstellung von 6-Amino-1,2-dihydro-1-hydroxy-2-imino-4-piperidino-pyrimidin

In dem Gemisch von 10 ml Äthanol und 4 ml n Natriumhydroxydlösung werden 0,5 g des gemäss Abschnitt a) hergestellten 6-Acetamido-1,2-dihydro-1-hydroxy-2-imino-4-piperidino-pyrimidinsgelöst. Die Lösung wird 30 Minuten lang gekocht, dann im Vakuum eingedampft, und der Rückstand wird in 10 ml Wasser ser aufgenommen. Die Kristalle werden abfiltriert, mit Wasser gewaschen, dann getrocknet. So erhält man 0,35 g (85 %) der Titelverbindung, die bei Vermischen mit dem Produkt gemäss Beispiel I keine Schmelzpunktdepression ergibt.

Beispiel VII

Herstellung von 6-Amino-1,2-dihydro-1-hydroxy-2-imino-4-piperidino-pyrimidin

a) Herstellung von 6-Acetamido-1,2-dihydro-1-acetoxy-2-imino-4-piperidino-pyrimidin

In dem Gemisch von 20 ml Acetonitril und 0,5 ml Piperidin werden 0,5 g (0,0013 Mol) 6-Acetamido-1,2-dihydro-1-acetoxy-2-imino-4-(p-toluol-sulfonyloxy)-pyrimidin bei Raumtemperatur 3 Stunden lang gerührt. Dann wird das Gemisch bei verringertem Druck eingedampft, und zum Rückstand werden 30 ml Äther gegeben. Der entstandene weisse kristalline Stoff wird abfiltriert, erst mit Äther, dann mit Wasser gewaschen und getrocknet.

So erhält man 0,24 g (64 %) der Titelverbindung.

Schmelzpunkt: 217 - 218 °C (zerfällt).

IR-Spektrum (KBr): 1710, 1680, 1630, 1570, 1530 $cm^{-1}$

UV-Spektrum (EtOH): 241, 293, 323 nm

NMR-Spektrum ($CDCl_3$ + TFA-d): 1,76 (m, 6H), 2,43 (s, 3H), 2,57 (s, 3H), 3,80 (m, 4H), 7,55 (s, 1H).

b) Herstellung von 6-Amino-1,2-dihydro-1-hydroxy-2-imino-4-piperidino-pyrimidin

In dem Gemisch von 20 ml Äthanol und 5 ml n Natriumhydroxydlösung werden 1,0 g (3,4 mMol) des gemäss Abschnitt a) hergestellten 6-Acetamido-1,2-dihydro-1-acetoxy-2-imino-4-piperidino-pyrimidins gelöst. Die Lösung wird 30 Minuten lang gekocht, dann im Vakuum eingedampft, Der Rückstand wird in 10 ml Wasser aufgenommen, die Kristalle werden abfiltriert, mit Wasser gewaschen und getrocknet.

So erhält man 0,54 g (76 %) der Titelverbindung, die bei Vermischen mit dem Produkt gemäss Beispiel I keine Schmelzpunktdepression ergibt.

Beispiel VIII

Herstellung von 6-Amino-1,2-dihydro-1-hydroxy-2-imino-4-piperidino-pyrimidin

2,0 g (0,0093 Mol) 6-Amino-1,2-dihydro-1-propionyloxy-2-imino-4-chlorpyrimidin in 10 ml Wasser werden in Gegenwart von 5 ml Piperidin eine halbe Stunde lang gekocht, dann werden 10 ml 1 molare wäßrige Natriumhydroxydlösung und 5 ml Wasser zugegeben. Nach einer weiteren halben Stunde Kochen wird die Lösung abgekühlt. Die Kristalle werden nach einstündigem Stehen abfiltriert und mit Wasser gewaschen.

So erhält man 1,36 g (83%) der Titelverbindung.

Beispiel IX

Herstellung von 6-Amino-2-imino-1-hydroxy-4-piperidino-1,2-dihydro-pyrimidin

In einem mit Rührer und Thermometer ausgestatteten Rundkolben werden unter Rühren und Kühlen mit Eiswasser 40,8 g (0,1 Mol) rohes 6-Acetamido-1-acetoxy-2-imino-4-mesitylen-sulfonyloxy-1,2-dihydro-pyrimidin mit 380 ml (327 g; 3,84 Mol) Piperidin umgesetzt. Das Rühren wird nach Erwärmen des Reaktionsgemisches auf Raumtemperatur fortgesetzt, bis chromatographisch nur noch die Produkte wahrgenommen werden können. Dies entspricht einer Zeit von etwa 24 Stunden. Dann wird das Piperidin in einem Bad mit einer Temperatur von 60 °C im Vakuum abdestilliert, und zum Rückstand werden 250 ml Wasser gegeben. Der abgesonderte Stoff wird nach dem Abkühlen abfiltriert und mit Wasser gewaschen, dann auf dem Filter getrocknet. Aus dem auf dem Filter verbliebenen festen Stoff wird das als Nebenprodukt enstandene Sulfonsäureamid mit wenig Toluol herausgewaschen. Durch das Trockendestillieren der Toluollösung gewinnt man 3,9 g (14,5 %) Mesitylen-sulfonyl-piperidid. Die auf dem Filter verbliebenen 13 g Stoff sind das gewünschte Produkt.

Die Mutterlauge wird weiter aufgearbeitet. Man setzt ihr 38 ml 10 gew.-%-ige wäßrige Natriumhydroxydlösung zu und destilliert im Vakuum, bis sie ölartig wird. So wird das noch vorhandene Piperidin entfernt. Dann werden 100 ml Wasser zugegeben, und der pH-Wert der Lösung wird mit 10 gew.-%-iger Salzsäure auf 7 eingestellt. Nach längerem Kühlen wird der abgesonderte Stoffabfiltriert und mit Wasser gewaschen. So erhält man weitere 5,3 g des Stoffes. Insgesamt werden 17,9 g (85,5 %) 6-Amino-2-imino-1-hydroxy-4-piperidino-1,2-dihydro-pyrimidin hergestellt. Schmelzpunkt: 240 - 260 °C (Zersetzung).

Die chromatographischen und spektroskopischen Eigenschaften des Produktes stimmen mit den in den vorhergehenden Beispielen I bis VIII angegebenen überein.

**Patentansprüche**

**Patentansprüche für folgende Vertragsstaaten: BE, CH, DE, FR, GB, IT, LI, NL, SE**

1. Pyrimidinderivate der allgemeinen Formel(n)

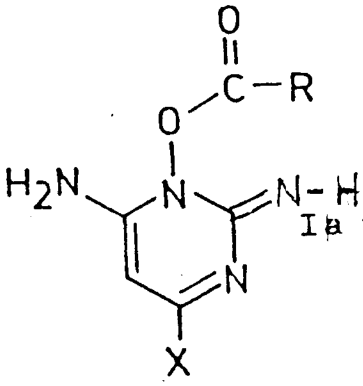

Ia und/oder

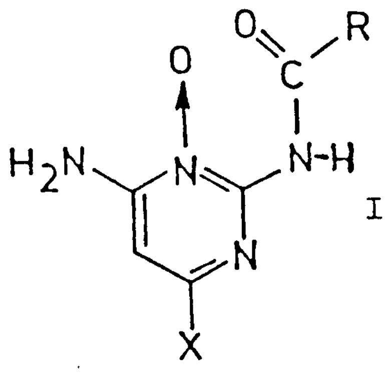

Ib und/oder

und/oder

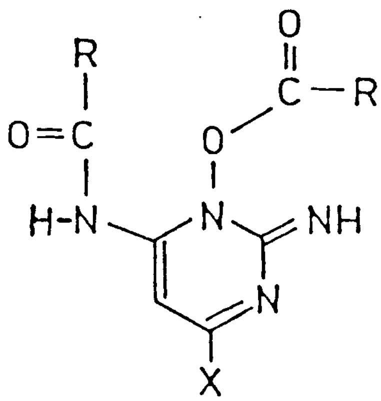

Ic,

worin

R für einen Alkylrest mit 1 bis 6 Kohlenstoffatom(en) oder einen, gegebenenfalls durch 1 oder mehr Halogenatom(e) substituierten, Arylrest mit 6 bis 12 Kohlenstoffatomen steht und

X Chlor oder Brom oder einen, gegebenenfalls 1- bis 3-fach substituierten, Arylsulfonyloxyrest mit einem Arylteil mit 6 bis 12 Kohlenstoffatomen bedeutet.

2. Pyrimidinderivate nach Anspruch 1, dadurch gekennzeichnet, daß der Alkylrest für den R stehen kann, ein solcher mit 1 bis 4 Kohlenstoffatomen ist.

3. Pyrimidinderivate nach Anspruch 1, dadurch gekennzeichnet, daß der Alkylrest für den R stehen kann, ein solcher mit 1 oder 2 Kohlenstoffatomen ist.

4. Pyrimidinderivate nach Anspruch 1, dadurch gekennzeichnet, daß der Arylrest, für den R stehen kann, ein Phenyl- oder Naphthylrest ist.

5. Pyrimidinderivate nach Anspruch 1 oder 4, dadurch gekennzeichnet, daß das bzw. die Halogenatom(e), durch welche(s) der Arylrest, für den R stehen kann, substituiert sein kann, [ein] Chlor- und/oder Bromatom(e) ist bzw. sind.

6. Pyrimidinderivate nach Anspruch 1 bis 4, dadurch gekennzeichnet, daß der Arylsulfonyloxyrest, für den X stehen kann, ein Phenylsulfonyloxy- oder Naphthylsulfonyloxyrest ist.

7. Pyrimidinderivate nach Anspruch 1 bis 6, dadurch gekennzeichnet, daß der bzw. die Substituent(en) am Arylring des Arylsulfonyloxyrestes, für den X stehen kann, [ein] Alkylrest(e) mit 1 bis 4 Kohlenstoffatom(en) ist bzw. sind.

**8.** Pyrimidinderivate nach Anspruch 1 bis 7, dadurch gekennzeichnet, daß der bzw. die Substituent(en) am Arylring des Arylsulfonyloxyrestes, für den X stehen kann, [ein] Alkylrest(e) mit 1 oder 2 Kohlenstoffatom(en) ist bzw. sind.

**9.** Pyrimidinderivate nach Anspruch 7, dadurch gekennzeichnet, daß der Substituent am Arylring des Arylsulfonyloxyrestes, für den X stehen kann, eine Methylgruppe zur Bildung des Tosyloxyrestes oder drei Methylgruppen zur Bildung des Mesitylensulfonyloxyrestes ist bzw. sind.

**10.** Verfahren zur Herstellung der Verbindungen nach Anspruch 1 bis 9 und/oder von Pyrimidinderivaten der allgemeinen Formel

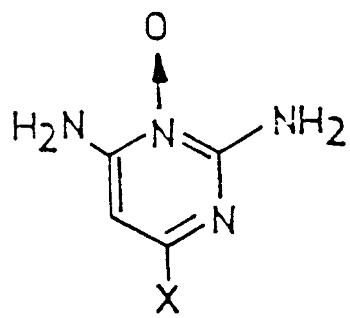

Id ,

worin

X die im Anspruch 1 oder 6 bis 9 angegebenen Bedeutungen hat,

durch Umsetzung von 2,6-Di-(amino)-pyrimidinderivaten der allgemeinen Formel

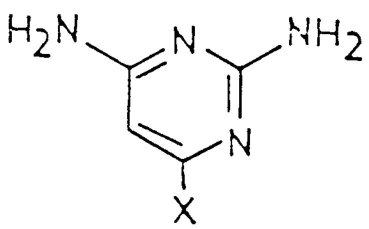

II ,

worin

X die im Anspruch 1 oder 6 bis 9 angegebenen Bedeutungen hat,

mit Percarbonsäure, dadurch gekennzeichnet, daß man die Umsetzung mit Säureanhydriden der allgemeinen Formel

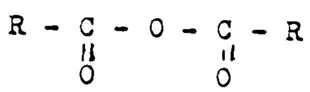

III ,

worin

R die in den Ansprüchen 1 bis 5 angegebenen Bedeutungen hat,

in Gegenwart von Wasser und Wasserstoffperoxyd durchführt und das erhaltene Produktgemisch oder die reinen pyrimidinderivate der allgemeinen Formel Ia, Ib, Ic beziehungsweise Id abtrennt und gegebenenfalls die reinen Pyrimidinderivate der allgemeinen Formel Ia, Ib beziehungsweise Ic beziehungsweise Gemische derselben hydrolysiert und/oder gegebenenfalls die Pyrimidinderivate der allgemeinen Formel Id mit Säureanhydriden der allgemeinen Formel III,

worin

R die in den Ansprüchen 1 bis 5 angegebenen Bedeutungen hat,

umsetzt.

**11.** Verfahren nach Anspruch 10, dadurch gekennzeichnet, daß man je Mol 2,6-Di-(amino)-pyrimidinderivat der allgemeinen Formel II 2 bis 20 Mol Wasser einsetzt.

**Patentansprüche für folgende Vertragsstaaten: AT, ES, GR**

**1.** Verfahren zur Herstellung von Pyrimidinderivaten der allgemeinen Formel(n)

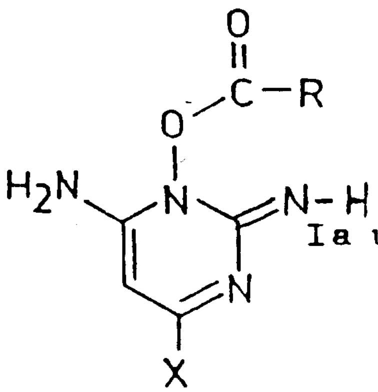
Ia und/oder

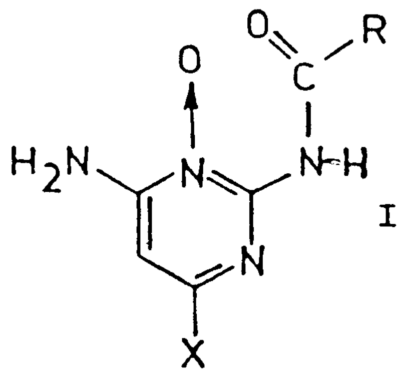
Ib und/oder

und/oder

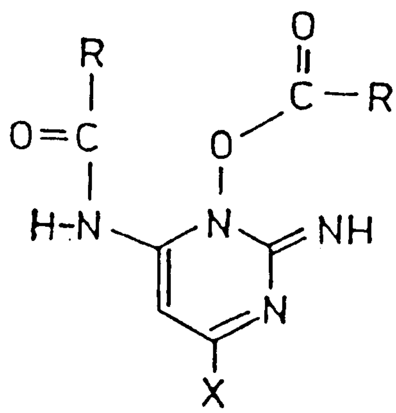
Ic,

worin

R für einen Alkylrest mit 1 bis 6 Kohlenstoffatom(en) oder einen, gegebenenfalls durch 1 oder mehr Halogenatom(e) substituierten, Arylrest mit 6 bis 12 Kohlenstoffatomen steht und

X Chlor oder Brom oder einen, gegebenenfalls 1- bis 3-fach substituierten, Arylsulfonyloxyrest mit einem Arylteil mit 6 bis 12 Kohlenstoffatomen bedeutet, und/oder von Pyrimidinderivaten der allgemeinen Formel

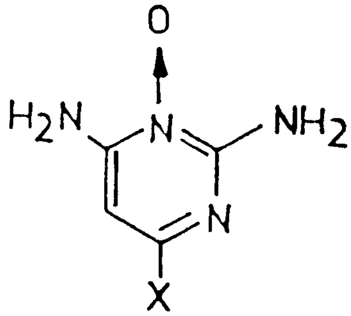
Id ,

worin

X die oben angegebenen Bedeutungen hat,
durch Umsetzung von 2,6-Di-(amino)-pyrimidinderivaten der allgemeinen Formel

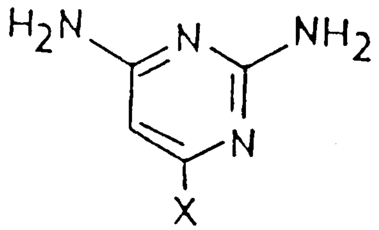

II ,

worin
X die oben angegebenen Bedeutungen hat,
mit Percarbonsäure, dadurch gekennzeichnet, daß man die Umsetzung mit Säureanhydriden der allgemeinen Formel

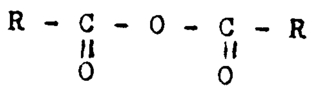

III ,

worin
R die oben angegebenen Bedeutungen hat,
in Gegenwart von Wasser und Wasserstoffperoxyd durchführt und das erhaltene Produktgemisch oder die reinen Pyrimidinderivate der allgemeinen Formel Ia, Ib, Ic beziehungsweise Id abtrennt und gegebenenfalls die reinen Pyrimidinderivate der allgemeinen Formel Ia, Ib beziehungsweise Ic beziehungsweise Gemische derselben hydrolysiert und/oder gegebenenfalls die Pyrimidinderivate der allgemeinen Formel Id mit Säureanhydriden der allgemeinen Formel III,

worin
R die oben angegebenen Bedeutungen hat,
umsetzt.

**2.** Verfahren nach Anspruch 1, dadurch gekennzeichnet, daß man Pyrimidinderivate, bei welchen der Alkylrest, für den R stehen kann, ein solcher mit 1 bis 4 Kohlenstoffatom(en) ist, herstellt.

**3.** Verfahren nach Anspruch 1, dadurch gekennzeichnet, daß man Pyrimidinderivate, bei welchen der Alkylrest, für den R stehen kann, ein solcher mit 1 oder 2 Kohlenstoffatom(en) ist, herstellt.

**4.** Verfahren nach Anspruch 1, dadurch gekennzeichnet, daß man Pyrimidinderivate, bei welchen der Arylrest, für den R stehen kann, ein Phenyl- oder Naphthylrest ist, herstellt.

**5.** Verfahren nach Anspruch 1 oder 4, dadurch gekennzeichnet, daß man Pyrimidinderivate, bei welchen das beziehungsweise die Halogenatom(e), durch welche[s] der Arylrest, für den R stehen kann, substituiert sein kann, [ein] Chlor- und/oder Bromatom(e) ist beziehungsweise sind, herstellt.

**6.** Verfahren nach Anspruch 1 bis 4, dadurch gekennzeichnet, daß man Pyrimidinderivate, bei welchen der Arylsulfonyloxyrest, für den X stehen kann, ein Phenylsulfonyloxy- oder Naphthylsulfonyloxyrest ist, herstellt.

**7.** Verfahren nach Anspruch 1 bis 5, dadurch gekennzeichnet, daß man Pyrimidinderivate, bei welchen der beziehungsweise die Substituent(en) am Arylring des Arylsulfonyloxyrestes, für den X stehen kann, [ein] Alkylrest(e) mit 1 bis 4 Kohlenstoffatom(en) ist beziehungsweise sind, herstellt.

**8.** Verfahren nach Anspruch 1 bis 7, dadurch gekennzeichnet, daß man Pyrimidinderivate, bei welchen der beziehungsweise die Substituent(en) am Arylring des Arylsulfonyloxyrestes, für den X stehen kann,

[ein] Alkylrest(e) mit 1 oder 2 Kohlenstoffatom(en) ist beziehungsweise sind, herstellt.

9. Verfahren nach Anspruch 7, dadurch gekennzeichnet, daß man Pyrimidinderivate, bei welchen der Substituent am Arylring des Arylsulfonyloxyrestes, für den X stehen kann, eine Methylgruppe zur Bildung des Tosyloxyrestes oder drei Methylgruppen zur Bildung des Mesitylensulfonyloxyrestes ist bzw. sind, herstellt.

10. Verfahren nach Anspruch 1, dadurch gekennzeichnet, daß man je Mol 2,6-Di-(amino)-pyrimidinderivat der allgemeinen Formel II 2 bis 20 Mol Wasser einsetzt.

**Claims**
**Claims for the following Contracting States: BE, CH, DE, FR, GB, IT, LI, NL, SE**

1. Pyrimidine derivatives of the general formula(e)

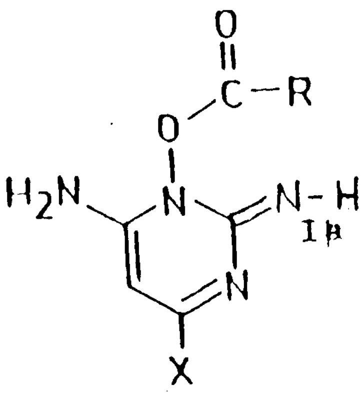
Ia and/or

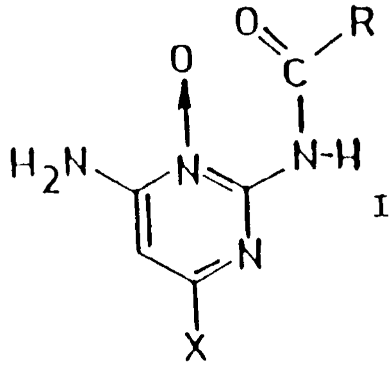
Ib and/or

and/or

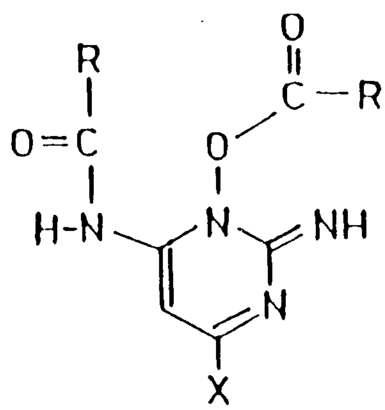
Ic,

wherein

R is an alkyl radical having from 1 to 6 carbon atoms or an aryl radical having from 6 to 12 carbon atoms and optionally substituted by one or more halogen atoms, and

X is chlorine or bromine or an optionally mono- to tri-substituted arylsulphonyloxy radical having an aryl moiety containing from 6 to 12 carbon atoms.

2. Pyrimidine derivatives according to claim 1, characterised in that the alkyl radical which R may represent has from 1 to 4 carbon atoms.

3. Pyrimidine derivatives according to claim 1, characterised in that the alkyl radical which R may represent has 1 or 2 carbon atoms.

4. Pyrimidine derivatives according to claim 1, characterised in that the aryl radical which R may represent is a phenyl or naphthyl radical.

**5.** Pyrimidine derivatives according to claim 1 or 4, characterised in that the halogen atom(s) which may be substituent(s) of the aryl radical which R may represent is(are) (a) chlorine and/or bromine atom(s).

**6.** Pyrimidine derivatives according to claims 1 to 4, characterised in that the arylsulphonyloxy radical which X may represent is a phenylsulphonyloxy or naphthylsulphonyloxy radical.

**7.** Pyrimidine derivatives according to claims 1 to 6, characterised in that the substituent(s) at the aryl ring of the arylsulphonyloxy radical which X may represent is(are) (an) alkyl radical(s) having from 1 to 4 carbon atoms.

**8.** Pyrimidine derivatives according to claims 1 to 7, characterised in that the substituent(s) at the aryl ring of the arylsulphonyloxy radical which X may represent is(are) (an) alkyl radical(s) having 1 or 2 carbon atoms.

**9.** Pyrimidine derivatives according to claim 7, characterised in that the substituent at the aryl ring of the arylsulphonyloxy radical which X may represent is a methyl group for the formation of the tosyloxy radical or three methyl groups for the formation of the mesitylenesulphonyloxy radical.

**10.** A process for the preparation of the compounds according to claims 1 to 9 and/or of pyrimidine derivatives of the general formula

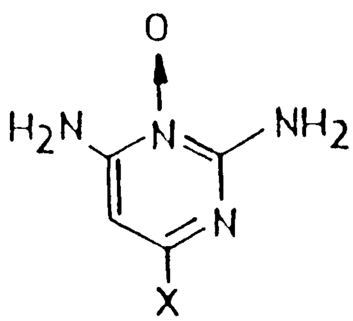

Id,

wherein

X has the meanings given in claim 1 or 6 to 9,

by reaction of 2,6-di-(amino)-pyrimidine derivatives of the general formula

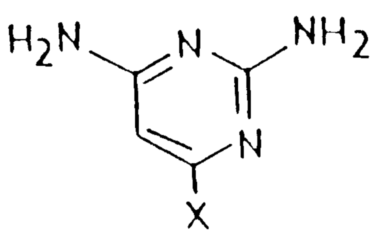

II

wherein

X has the meanings given in claim 1 or 6 to 9,

with percarboxylic acid, characterised in that the reaction is carried out with acid anhydrides of the general formula

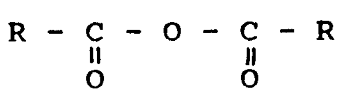

III,

wherein

R has the meanings given in claims 1 to 5,

in the presence of water and hydrogen peroxide, and the resulting product mixture or the pure

pyrimidine derivatives of the general formula Ia, Ib, Ic or Id is/are separated and optionally the pure pyrimidine derivatives of the general formula Ia, Ib or Ic or mixtures thereof are hydrolysed and/or optionally the pyrimidine derivatives of the general formula Id are reacted with acid anhydrides of the general formula III,

wherein

R    has the meanings given in claims 1 to 5.

**11.** A process according to claim 10, characterised in that from 2 to 20 moles of water are used per mole of 2,6-di-(amino)-pyrimidine derivative of the general formula II.

**Claims for the following Contracting States: AT, ES, GR**

**1.** A process for the preparation of pyrimidine derivatives of the general formula(e)

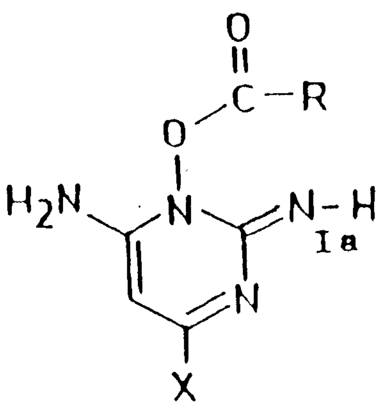

Ia and/or

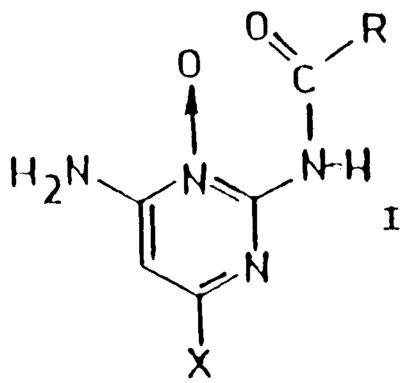

Ib and/or

,

and/or

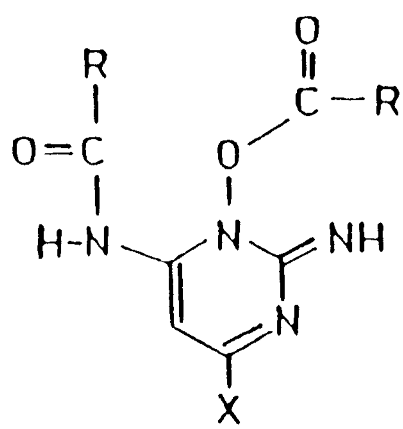

Ic,

wherein

R    is an alkyl radical having from 1 to 6 carbon atoms or an aryl radical having from 6 to 12 carbon atoms and optionally substituted by one or more halogen atoms, and

X    is chlorine or bromine or an optionally mono- to tri-substituted arylsulphonyloxy radical having an aryl moiety containing from 6 to 12 carbon atoms,

and/or of pyrimidine derivatives of the general formula

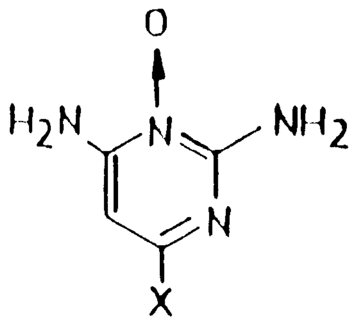

Id,

wherein

X has the meanings given above,

by reaction of 2,6-di-(amino)-pyrimidine derivatives of the general formula

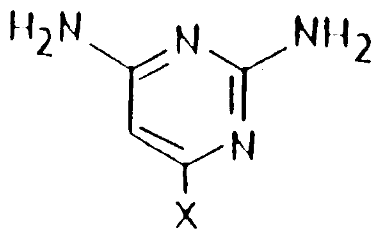

II

wherein

X has the meanings given above,

with percarboxylic acid, characterised in that the reaction is carried out with acid anhydrides of the general formula

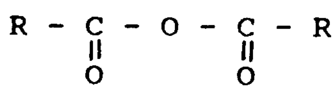

III,

wherein

R has the meanings given above,

in the presence of water and hydrogen peroxide, and the resulting product mixture or the pure pyrimidine derivatives of the general formula Ia, Ib, Ic or Id are separated and optionally the pure pyrimidine derivatives of the general formula Ia, Ib or Ic or mixtures thereof are hydrolysed and/or optionally the pyrimidine derivatives of the general formula Id are reacted with acid anhydrides of the general formula III,

wherein

R has the meanings given above.

**2.** A process according to claim 1, characterised in that there are prepared pyrimidine derivatives wherein the alkyl radical which R may represent has from 1 to 4 carbon atoms.

**3.** A process according to claim 1, characterised in that there are prepared pyrimidine derivatives wherein the alkyl radical which R may represent has 1 or 2 carbon atoms.

**4.** A process according to claim 1, characterised in that there are prepared pyrimidine derivatives wherein the aryl radical which R may represent is a phenyl or naphthyl radical.

**5.** A process according to claim 1 or 4, characterised in that there are prepared pyrimidine derivatives wherein the halogen atom(s) which may be substituent(s) of the aryl radical which R may represent is-(are) (a) chlorine and/or bromine atom(s).

6. A process according to claims 1 to 4, characterised in that there are prepared pyrimidine derivatives wherein the arylsulphonyloxy radical which X may represent is a phenylsulphonyloxy or naphthylsulphonyloxy radical.

7. A process according to claims 1 to 5, characterised in that there are prepared pyrimidine derivatives wherein the substituent(s) at the aryl ring of the arylsulphonyloxy radical which X may represent is(are) (an) alkyl radical(s) having from 1 to 4 carbon atoms.

8. A process according to claims 1 to 7, characterised in that there are prepared pyrimidine derivatives wherein the substituent(s) at the aryl ring of the arylsulphonyloxy radical which X may represent is(are) (an) alkyl radical(s) having 1 or 2 carbon atoms.

9. A process according to claim 7, characterised in that there are prepared pyrimidine derivatives wherein the substituent at the aryl ring of the arylsulphonyloxy radical which X may represent is a methyl group for the formation of the tosyloxy radical or three methyl groups for the formation of the mesitylenesulphonyloxy radical.

10. A process according to claim 1, characterised in that from 2 to 20 moles of water are used per mole of 2,6-di-(amino)-pyrimidine derivative of the general formula II.

**Revendications**
**Revendications pour les Etats contractants suivants: BE, CH, DE, FR, GB, IT, LI, NL, SE**

1. Dérivés de pyrimidine de formule(s) générale(s)

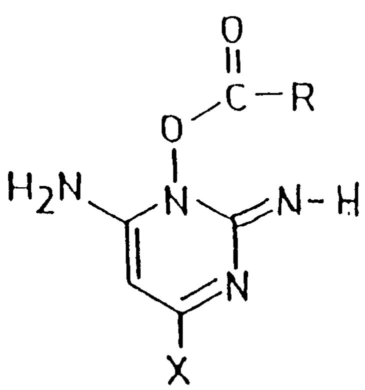

(Ia) et/ou

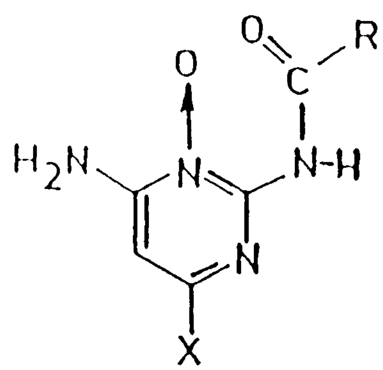

(Ib)

et/ou

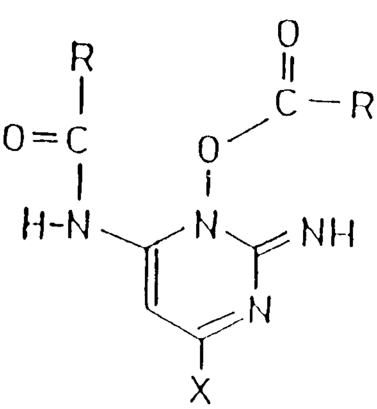

(Ic)

dans lesquelles

R représente un reste alcoyle comportant 1 à 16 atome(s) de carbone, ou un reste aryle, éventuellement substitué par un ou plusieurs atome(s) d'halogène, comportant 6 à 12 atomes de carbone, et

X représente un atome de chlore ou de brome, ou un reste arylsulfonyloxy, éventuellement substitué 1 à 3 fois, avec un motif aryle comportant 6 à 12 atomes de carbone.

2. Dérivés de pyrimidine selon la revendication 1, caractérisés en ce que le reste alcoyle qui peut être représenté par R est un reste alcoyle comportant 1 à 4 atome(s) de carbone.

3. Dérivés de pyrimidine selon la revendication 1, caractérisés en ce que le reste alcoyle qui peut être représenté par R est un reste alcoyle comportant 1 ou 2 atome(s) de carbone.

4. Dérivés de pyrimidine selon la revendication 1, caractérisés en ce que le reste aryle qui peut être représenté par R est un reste phényle ou naphtyle.

5. Dérivés de pyrimidine selon la revendication 1 ou 4, caractérisés en ce que le ou les atome(s) d'halogène, au moyen duquel/desquels le reste aryle, pouvant être représenté par R, peut être substitué, est/sont un ou des atome(s) de chlore et/ou de brome.

6. Dérivés de pyrimidine selon l'une des revendications 1 à 4, caractérisés en ce que le reste arylsulfonyloxy, qui peut être représenté par X, est un reste phénylsulfonyloxy ou naphtylsulfonyloxy.

7. Dérivés de pyrimidine selon l'une des revendications 1 à 6, caractérisés en ce que le ou les substituant(s) du cycle d'aryle du reste arylsulfonyloxy, qui peut être représenté par X, est/sont un ou des reste(s) alcoyle comportant 1 à 4 atome(s) de carbone.

8. Dérivés de pyrimidine selon l'une des revendications 1 à 7, caractérisés en ce que le ou les substituant(s) du cycle d'aryle du reste arylsulfonyloxy, qui peut être représenté par X, est/sont un ou des reste(s) alcoyle comportant 1 ou 2 atome(s) de carbone.

9. Dérivés de pyrimidine selon la revendication 7, caractérisés en ce que le ou les substituant(s) du cycle d'aryle du reste arylsulfonyloxy, qui peut être représenté par X, est un groupe méthyle pour former le reste tosyloxy, ou sont trois groupes méthyle pour former le reste mésitylènesulfonyloxy.

10. Procédé de préparation des composés selon l'une des revendications 1 à 9 et/ou des dérivés de pyrimidine répondant à la formule générale

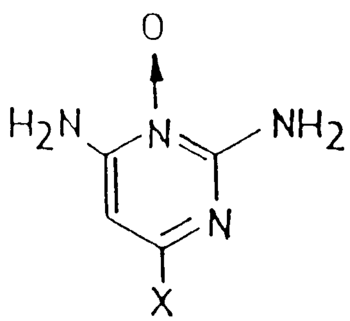

(Id)

dans laquelle

X a les significations indiquées dans la revendication 1 ou dans l'une des revendications 6 à 9,

en faisant réagir des dérivés 2,6-di-(amino)-pyrimidine répondant à la formule générale

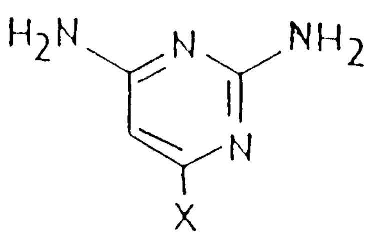

(II)

dans laquelle

X a les significations indiquées dans la revendication 1 ou dans l'une des revendications 6 à 9,

avec de l'acide percarbonique, caractérisé en ce qu'on réalise la réaction avec des anhydrides d'acides répondant à la formule générale

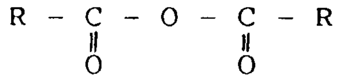

(III)

dans laquelle

R a les significations indiquées dans les revendications 1 à 5,

en présence d'eau et de peroxyde d'hydrogène, et on sépare le mélange de produits obtenu ou les dérivés de pyrimidine purs, répondant à l'une des formules Ia, Ib, Ic et Id, et éventuellement, on hydrolyse les dérivés de pyrimidine purs, répondant à l'une des formules Ia, Ib et Ic, ou des mélanges de ces dérivés, et/ou éventuellement on fait réagir les dérivés de pyrimidine de formule générale Id avec des anhydrides d'acides répondant à la formule générale III

dans laquelle

R a les significations indiquées dans les revendications 1 à 5.

**11.** Procédé selon la revendication 10, caractérisé en ce qu on utilise 2 à 20 moles d'eau par mole de dérivé 2,6-di-(amino)-pyrimidine répondant à la formule générale II.

**Revendications pour les Etats contractants suivants: AT, ES, GR**

**1.** Procédé de préparation de dérivés de pyrimidine de formule(s) générale(s)

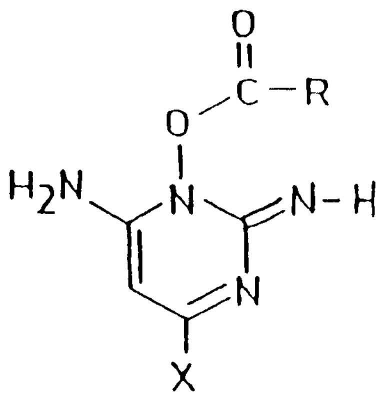

(Ia) et/ou

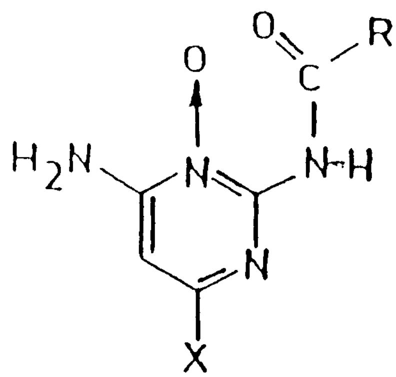

(Ib)

et/ou

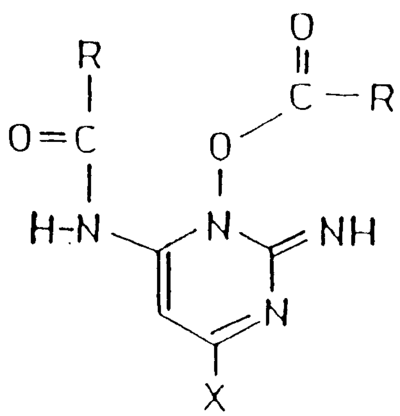

(Ic)

dans lesquelles

R représente un reste alcoyle comportant 1 à 16 atome(s) de carbone, ou un reste aryle, éventuellement substitué par un ou plusieurs atome(s) d'halogène, comportant 6 à 12 atomes de carbone, et

X représente un atome de chlore ou de brome, ou un reste arylsulfonyloxy, éventuellement

substitué 1 à 3 fois, avec un motif aryle comportant 6 à 12 atomes de carbone, et/ou de dérivés de pyrimidine répondant à la formule générale

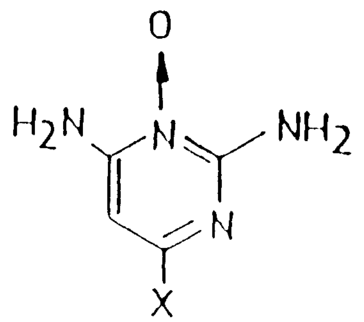

(Id)

dans laquelle

X a les significations indiquées ci-dessus,

en faisant réagir des dérivés 2,6-di-(amino)-pyrimidine répondant à la formule générale

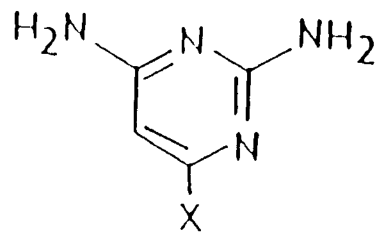

(II)

dans laquelle

X a les significations indiquées ci-dessus,

avec de l'acide percarbonique, caractérisé en ce qu'on réalise la réaction avec des anhydrides d'acides répondant à la formule générale

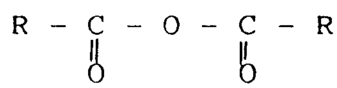

(III)

dans laquelle

R a les significations indiquées ci-dessus,

en présence d'eau et de peroxyde d'hydrogène, et on sépare le mélange de produits obtenu ou les dérivés de pyrimidine purs, répondant à l'une des formules Ia, Ib, Ic et Id, et éventuellement, on hydrolyse les dérivés de pyrimidine purs, répondant à l'une des formules Ia, Ib et Ic, ou des mélanges de ces dérivés, et/ou éventuellement on fait réagir les dérivés de pyrimidine de formule générale Id avec des anhydrides d'acides répondant à la formule générale III

dans laquelle

R a les significations indiquées ci-dessus.

2. Procédé selon la revendication 1, caractérisé en ce qu'on prépare des dérivés de pyrimidine dans lesquels le reste alcoyle, qui peut être représenté par R, est un reste alcoyle comportant 1 à 4 atome-(s) de carbone.

3. Procédé selon la revendication 1, caractérisé en ce qu'on prépare des dérivés de pyrimidine dans lesquels le reste alcoyle, qui peut être représenté par R, est un reste alcoyle comportant 1 ou 2 atome-(s) de carbone.

4. Procédé selon la revendication 1, caractérisé en ce qu'on prépare des dérivés de pyrimidine dans lesquels le reste aryle, qui peut être représenté par R, est un reste phényle ou naphtyle.

**5.** Procédé selon la revendication 1 ou 4, caractérisé en ce qu'on prépare des dérivés de pyrimidine dans lesquels le ou les atome(s) d'halogène, au moyen duquel/desquels le reste aryle, pouvant être représenté par R, peut être substitué, est/sont un ou des atome(s) de chlore et/ou de brome.

**6.** Procédé selon l'une des revendications 1 à 4, caractérisé en ce qu'on prépare des dérivés de pyrimidine dans lesquels le reste arylsulfonyloxy, qui peut être représenté par X, est un groupe phénylsulfonyloxy ou naphtylsulfonyloxy.

**7.** Procédé selon l'une des revendications 1 à 5, caractérisé en ce qu'on prépare des dérivés de pyrimidine dans lesquels le ou les substituant(s) du cycle d'aryle du reste arylsulfonyloxy, qui peut être représenté par X, est/sont un ou des reste(s) alcoyle comportant 1 à 4 atome(s) de carbone.

**8.** Procédé selon l'une des revendications 1 à 7, caractérisé en ce qu on prépare des dérivés de pyrimidine dans lesquels le ou les substituant(s) du cycle d'aryle du reste arylsulfonyloxy, qui peut être représenté par X, est/sont un ou des reste(s) alcoyle comportant 1 ou 2 atome(s) de carbone.

**9.** Procédé selon la revendication 7, caractérisé en ce qu'on prépare des dérivés de pyrimidine dans lesquels le ou les substituant(s) du cycle d'aryle du reste arylsulfonyloxy, qui peut être représenté par X, est un groupe méthyle pour former le reste tosyloxy, ou sont trois groupes méthyle pour former le reste mésitylènesulfonyloxy.

**10.** Procédé selon la revendication 1, caractérisé en ce qu'on utilise 2 à 20 moles d'eau par mole de dérivé 2,6-di-(amino)-pyrimidine répondant à la formule générale II.